# EUROPÄISCHE PATENTSCHRIFT

(11) **EP 2 872 876 B1**
(45) Veröffentlichungstag und Bekanntmachung des Hinweises auf die Patenterteilung: **19.02.2020**
(21) Anmeldenummer: 13720744.5
(22) Anmeldetag: 27.03.2013
(51) Int. Cl.: G01N 21/64, G01N 31/22, G01N 21/77, G01N 33/497

(54) **OPTO-CHEMISCHER SENSOR UND DESSEN VERWENDUNG**
OPTO-CHEMICAL SENSOR AND ITS USE
CAPTEUR OPTO-CHIMIQUE ET SON UTILISATION

(30) Priorität: 30.03.2012 AT 3892012
(43) Veröffentlichungstag der Anmeldung: 20.05.2015
(73) Patentinhaber: Joanneum Research Forschungsgesellschaft mbH, 8010 Graz (AT)
(72) Erfinder: WOLF, Christian, A-8010 Graz (AT)
(74) Vertreter: Dilg, Haeusler, Schindelmann Patentanwaltsgesellschaft mbH
(86) Internationale Anmeldenummer: PCT/AT2013/000051
(87) Internationale Veröffentlichungsnummer: WO 2013/142886

(56) Entgegenhaltungen:
- WO-A1-00/16074
- XIANYAN WANG ET AL: "Electrospun Nanofibrous Membranes for Highly Sensitive Optical Sensors", NANO LETTERS, Bd. 2, Nr. 11, 1. November 2002 (2002-11-01), Seiten 1273-1275, XP055071364, ISSN: 1530-6984, DOI: 10.1021/nl020216u
- SHENGYANG TAO ET AL: "Fluorescent nanofibrous membranes for trace detection of TNT vapor", JOURNAL OF MATERIALS CHEMISTRY, Bd. 17, Nr. 26, 1. Januar 2007 (2007-01-01), Seite 2730, XP055071374, ISSN: 0959-9428, DOI: 10.1039/b618122h
- JIRI MOSINGER ET AL: "Fluorescent Polyurethane Nanofabrics: A Source of Singlet Oxygen and Oxygen Sensing", LANGMUIR, Bd. 26, Nr. 12, 15. Juni 2010 (2010-06-15) , Seiten 10050-10056, XP055071377, ISSN: 0743-7463, DOI: 10.1021/la1001607
- MICHELA QUARANTA ET AL: "Indicators for optical oxygen sensors", BIOANALYTICAL REVIEWS, Bd. 4, Nr. 2-4, 24. November 2012 (2012-11-24), Seiten 115-157, XP055071464, ISSN: 1867-2086, DOI: 10.1007/s12566-012-0032-y
- ILARIA CUCCHI ET AL: "Fluorescent Electrospun Nanofibers Embedding Dye-Loaded Zeolite Crystals", SMALL, Bd. 3, Nr. 2, 5. Februar 2007 (2007-02-05) , Seiten 305-309, XP055071367, ISSN: 1613-6810, DOI: 10.1002/smll.200600472
- JOSÉ M. MORAN-MIRABAL ET AL: "Electrospun Light-Emitting Nanofibers", NANO LETTERS, Bd. 7, Nr. 2, 1. Februar 2007 (2007-02-01) , Seiten 458-463, XP055071631, ISSN: 1530-6984, DOI: 10.1021/nl062778+
- CUISONG ZHOU ET AL: "Electrospun Ru(bpy)32+-doped nafion nanofibers for electrochemiluminescence sensing", THE ANALYST, Bd. 135, Nr. 5, 1. Januar 2010 (2010-01-01), Seite 1004, XP055071633, ISSN: 0003-2654, DOI: 10.1039/b923031a

## Beschreibung

Die vorliegende Erfindung bezieht sich auf einen optochemischen Sensor sowie dessen Verwendung zur Bestimmung von Substanzen in der Gas- oder Flüssigphase. Der erfindungsgemäße optochemische Sensor umfasst eine auf einen Träger aufgebrachte aus mit einem Lumineszenzfarbstoff dotierten, gesponnenen Nanofasern gebildete Sensorschicht, deren Emissionsvermögen nach Anregung mit elektromagnetischer Strahlung durch nachzuweisende Substanzen, wie O₂, CO₂, Lactat, Glucose, NH₃, SO₂, H₂O₂, Stickoxide, halogenierte Kohlenwasserstoffe bzw. zu ermittelnde Messgrößen, wie dem pH-Wert, der Feuchte und der Temperatur in einer Gas- oder Flüssigphase veränderbar ist, wobei die Sensorschicht aus Nanofasern aus Polymeren mit einem Durchmesser zwischen 50 nm und 1000 nm gebildet ist, wobei die Nanofasern mit wenigstens einem Lumineszenzfarbstoff gewählt aus der Gruppe der Metallporphyrine, Benzoporphyrine, Azabenzoporphyrine, Napthoporphyrine, Phthallocyanine, Perylene, Perylendiimine, Pyrene; Xanthenfarbstoffe, Azofarbstoffe, Bodipyfarbstoffe, Azabodipyfarbstoffe, Cyaninfarbstoffe, Bipyridine, Bipyridyle, Phenantroline, Coumarine und Acetylacetonate von Ruthenium und Iridium; Acridinfarbstoffe, Oxazinfarbstoffe, Coumarine, Azaannulene, Squarine, 8-Hydroxychinoline, Polymethine, lumuneszenten Nanopartikel gewählt aus Quantenpunkten oder Nanokristallen; Carbostyryle, Terbiumkomplexe, anorganischen Phosphore oder mit lonophoren gewählt aus Kronenethern, verbundenen oder derivatisierten Farbstoffen der vorhin genannten Klassen dotiert sind, und wobei die Nanofasern weiterhin ein anionisches, kationisches oder nicht ionisches Tensid enthalten.

Der erfindungsgemäße optochemische Sensor ist in Anspruch 1 definiert.
Die erfindungsgemäße Verwendung des
optochemischen Sensors ist in Anspruch 15 definiert.

Sensoren zum Messen der Konzentration von bestimmten Substanzen, wie Gasen, Metaboliten oder Ionen in Lösungen bzw. Feststoffen arbeiten entweder elektrochemisch, wobei sie in diesem Fall den Nachteil aufweisen, dass sie für einen quantitativen Nachweis des zu bestimmenden Gases einen Teil der zu bestimmenden Substanz verbrauchen, wodurch das Messergebnis verfälscht wird oder aber es wurden in letzter Zeit opto-chemische Sensoren entwickelt, welche sich dadurch auszeichnen, dass sie die Zusammensetzung des Analyten über die Zeit nicht verändern, sondern dass sie den quantitativen Nachweis der Konzentration der zu bestimmenden Substanz bzw. des zu bestimmenden Gases nur durch eine Änderung der Lumineszenzeigenschaften, wie z.B. der Lumineszenzlöschung der in dem Sensor enthaltenen lumineszierenden Substanz anzeigen. Hierbei werden charakteristische Parameter, wie beispielsweise die Lumineszenzintensität, die Phasenverschiebung des Lumineszenzsignals oder auch die Abklingzeit der Lumineszenz überprüft und durch Vergleich des Lumineszenzsignals mit einer Kalibrierfunktion gelingt ein quantitativer Nachweis der zu messenden Substanz. Für opto-chemische Sensoren ist es erforderlich, dass die lumineszierenden Farbstoffe in eine feste Matrix, wie beispielsweise Polymere eingemischt werden, wobei während einer Messung einer zu vermessenden Substanz, diese durch die Polymermatrix zu den darin eingebetteten Farbstoffmolekülen diffundieren muss, damit die Fluoreszenzfarbstoffe beeinflusst werden.

X. Wang et al., Nano Lett., Vol. 2, Nr. 11, 2002, 1273 ff beschreiben die Entwicklung von empfindlichen Detektionstechniken, bei welchen nanogesponnene, optische Fluoreszenzsensoren hergestellt werden, welche im Vergleich zu Sensoren gemäß dem Stand der Technik eine größere Oberfläche und eine höhere Ansprechgeschwindigkeit aufweisen sollen, wobei diese Erhöhung ausschließlich durch Anwendung der Elektrospinntechnik erreicht wurde.

S. Tao et al., J MATER CHEM, 2007, 17, 2730 beschreibt die Herstellung von aus Tetraphenylporphyrin und Silane hergestellte Nanofasern mit reduziertem Durchmesser zur schnellen Detektion von puren Explosivstoffen.

J. Mosinger et al., Langmuir 2010, 26(12) 10050 ff beschreiben auf Polyurethan basierende Nanofasern verringerten Durchmessers, die durch ein Elektrospinverfahren hergestellt wurden und welche TPP-Beladungen aufweisen.

M. Quaranta et al., Bioanal Rev (2012) 4:115-157 beschreiben verschiedene Indikatoren für optische Sauerstoffsensoren sowie Kriterien für deren Verwendung.

I. Cucchi et al., Small 2007, 3, No. 2, 305 ff beschreiben elektrogesponnene Nanofasern, in welche mit Farbstoffen dotierte Zeolithwirkstoffe eingebettet wurden.

Die WO 00/16074 beschreibt einen opto-chemischen Sensor bestehend aus einer Polymermatrix und einem darin enthaltenen Lumineszenzfarbstoff, dessen Polymermatrix aus einem Polymer ohne Zusatz von Weichmachern gebildet ist.

In jüngerer Vergangenheit wurden hoch empfindliche, opto-chemische Sauerstoffsensoren entwickelt, bei welchen die Lumineszenzfarbstoffe in Membranen aus Nanofasern, welche durch Elektrospinnen hergestellt wurden, eingebettet sind. So ist beispielsweise aus dem Artikel von J. Wang et al., Chem.Commun., 2009, 5868 ff. ein derartiger Sauerstoffsensor bekannt geworden, bei welchem ein auf einem Cu(I)-Komplex basierender Farbstoff in eine aus Polystyrolmaterial bestehende Nanofasermembran eingebettet ist, welche Membran durch Elektrospinnen hergestellt wurde. Die Nanofasern bilden hierbei keine durchgehende Schicht aus, sondern eine Schicht, welche aus übereinander angeordneten, ungeordneten Polymerfäden besteht. Eine derartige Schicht weist einen porösen Aufbau auf und besitzt somit im Vergleich zu durchgehenden Polymermatrizes ein vergrößertes Oberflächen-zu-Volumen-Verhältnis, so dass es mit derartigen Schichten gelingt, die Zugänglichkeit des Analyten zu dem Farbstoff deutlich zu erhöhen, wodurch es gelungen ist, empfindlichere Sensoren zur Verfügung zu stellen.

Neben der Empfindlichkeit der Sensoren ist es jedoch in einer Vielzahl von Anwendungen erforderlich, nicht nur ein genaues Messsignal zu erhalten, sondern insbesondere den Messwert schnell zu erhalten, insbesondere in Echtzeit zur Verfügung zu stellen, um die Aussagekraft der Messungen und die Einsatzgebiete der Sensoren weiter zu erhöhen.

Die vorliegende Erfindung zielt nun darauf ab, ein Sensormaterial der eingangs genannten Art als einen opto-chemischen Sensor zur Verfügung zu stellen, welcher neben einer hohen Messgenauigkeit ein gegenüber herkömmlichen Sensoren deutlich verbessertes, insbesondere schnelleres Ansprechverhalten zur Verfügung stellt.

Zur Lösung dieser Aufgabe wird das Sensormaterial gemäß der Erfindung als ein opto-chemischer Sensor mit einer Ansprechzeit t₉₅ von unter 1 s verwendet. Indem der Durchmesser der Nanofasern zwischen 50 nm und 1000 nm gewählt wird, gelingt es, eine Sensorschicht zur Verfügung zu stellen, welche einen porösen Aufbau aufweist, die ein im Vergleich zu einer durchgehenden Sensorschicht bedeutend vergrößertes Oberflächen-zu-Volumen-Verhältnis aufweist, so dass während einer Messung die Diffusionswege der zu vermessenden Moleküle massiv herabgesetzt werden und somit einerseits die Messgenauigkeit erhöht wird und andererseits eine Reduktion der Ansprechzeiten erzielt wird. Indem weiterhin die Nanofasern mit wenigstens einem Lumineszenzfarbstoff gewählt aus der Gruppe Metallporphyrine, Benzoporphyrine, Azabenzoporphyrine, Napthoporphyrine, Phthallocyanine, polycyclische aromatischen Kohlenwasserstoffen, insbesondere Perylene, Perylendiimine, Pyrene; Xanthenfarbstoffe, Azofarbstoffe, Bodipyfarbstoffe, Azabodipyfarbstoffe, Cyaninfarbstoffe, Metall-Ligand-Komplexfarbstoffe, insbesondere Bipyridine, Bipyridyle, Phenantroline, Coumarine und Acetylacetonate von Ruthenium und Iridium; Acridinfarbstoffe, Oxazinfarbstoffe, Coumarine, Azaannulene, Squarine, 8-Hydroxychinoline, Polymethine, luminiszenten Nanopartikel, gewählt aus Quantenpunkten, Nanokristallen; Carbostyryle, Terbiumkomplexe, anorganischen Phosphoren oder mit lonophroen, gewählt aus Kronenethern verbundenen oder derivatisierten Farbstoffen der vorhin genannten Klassen dotiert sind, gelingt es in überraschender Weise, die Ansprechgeschwindigkeit des Sensors um ein Vielfaches gegenüber herkömmlichen Sensoren zu erhöhen.

Diese stark herabgesetzte Ansprechzeit von unter 1 s ist insbesondere deshalb überraschend, da vergleichbare Sensoren gemäß dem Stand der Technik, welche andere Lumineszenzfarbstoffe einsetzen, jedoch sonst analog hergestellt sind, bedeutend langsamere Ansprechzeiten aufweisen. Durch Einmischen von wenigstens einem Lumineszenzfarbstoff gewählt aus der genannten Gruppe gelingt es, die Ansprechzeiten t₉₅ eines opto-chemischen Sensors, welcher eine Sensorschicht aus Nanofasern, die mit derartigen Lumineszenzfarbstoffen dotiert sind, aufweist, auf deutlich unter 1 s abzusenken im Vergleich zu Ansprechzeiten von herkömmlichen Sensoren in der Größenordnung von wenigstens 3 s, insbesondere wenigstens 5 s.

Indem der verwendete opto-chemische Sensor so ausgebildet ist, dass die Nanofasern ein anionisches, kationisches oder nicht ionisches Tensid enthält, wird überraschenderweise festgestellt, dass die Ansprechzeiten noch weiter auf Werte von deutlich unter 1 s abgesenkt werden können. Das Einmischen eines Tensids in die Nanofasern bewirkt hierbei eine weitere Vergröberung der Oberfläche, insbesondere eine weitere Erhöhung der Porosität der Oberfläche der Nanofasern, so dass die Zugänglichkeit der Analyten zu dem Farbstoff weiter erhöht wird.

Falls nicht anders erwähnt, ist mit Ansprechzeit im Allgemeinen die Ansprechzeit t₉₅ gemeint, welche die Zeitdauer des Signalanstiegs von 0 % bis 95 % der endgültigen Signalintensität bzw. den Signalabfall von 100 % bis 5 % beschreibt.

Besonders kurze Ansprechzeiten von deutlich unter 1 s werden gemäß der vorliegenden Erfindung überraschenderweise dadurch erreicht, dass der wenigstens eine Lumineszenzfarbstoff aus Pd(II)-Octaethylporphyrin, Pt(II)-Octaethylporphyrin, Pd(II)-Tetraphenylporphyrin, Pt(II)-Tetraphenylporphyrin, Pd(II)-meso-Tetraphenyl-tetrabenzoporphyrin, Pt(II)-meso-Tetraphenyl-tetrabenzoporphyrin, Pd(II)-Octaethylporphyrinketon, Pt(II)-Octaethylporphyrinketon, Pd(II)-meso-Tetra(pentafluorphenyl) porphyrin, Pt(II)-meso-Tetra(pentafluorphenyl)porphyrin, Ru(II)-Tris(4,7-diphenyl-1,10-phenanthrolin) (Ru(dpp)3), Ru(II)-Tris(1,10-phenanthrolin) (Ru(phen)3), Tris(2,2-bipyridine)ruthenium(II)chlorid Hexahydrat [Ru(bpy)3], Erythrosine B, Fluorescein, Eosin, Iridium(III)((N-methyl-benzoimidazol-2-yl)-7-(diethylamino)-coumarin))2(acetylacetonat), Iridium(III)((benzothiazol-2-yl)-7-(diethylamino)-coumarin))2(acetylacetonat), Lumogenrot, Lumogengelb, Macrolex Fluoreszenz rot, Macrolex Fluoreszenz gelb, Rhodamin B, Rhodamin 6G, TAMRA, Texasrot, Sulfo-Rhodamin, m-Cresolrot (mCP), Thymolblau (TB), Xylenolblau (XB), Cresolrot (CR), Chlorphenolblau, Bromcresolgrün (BG), Bromcresolrot (BP), Bromthymol lau (BTB), 4-Nitrophenol (NP), Alizarin, Phenolphtalein (PP), o-Cresolphtalein (oCP), Chlorphenolrot (CPR), Calmagit (CG), Bromxylenolblau (BXB), Methylrot (MR), Phenolrot (PR), Neutralrot (NR), Nitrazingelb (NY), 3,4,5,6 Tetrabromphenolsulfonphtalein, Kongorot (CR), Fluorescein, Eosin, 2',7'-Dichlorfluorescein, 5(6)-Carboxy-fluorecsein, Carboxynaphtofluorescein, 8-Hydroxypyren-1,3,6-trisulfonsäure (HPTS), Seminaphthorhodafluors (SNARF Farbstoffe) Seminaphtofluorescein (SNAFL Farbstoffe) verwendet wird. Obwohl es sich bei dieser Gruppe von Lumineszenzfarbstoffen um gängige Lumineszenzfarbstoffe handelt, konnte durch Einmischen dieser Lumineszenzfarbstoffe in das Polymermaterial und anschließendes Elektrospinnen der Mischung zu den die Sensorschicht ausbildenden Nanofasern eine deutliche Herabsetzung der Ansprechzeit bzw. -dauer gegenüber beispielsweise Farbstoffen auf Europiumbasis erzielt werden.

Besonders feine Nanofasern und insbesondere gut verspinnbare Nanofasern werden gemäß einer Weiterbildung der Erfindung dadurch erhalten, dass ein Tensid aus der Gruppe bestehend aus anionischen Tensiden auf Basis von Carboxylaten, Sulfonaten und Sulfaten, gewählt aus Alkylcarboxylate, Alkylbenzolsulfonate, Natriumdodecylbenzolsulfonat, sekundäre Alkansulfonate, Sulfate gewählt aus Natriumlaurylsulfat, Dodecylsulfat, nichtionischen Tensiden auf Basis von Fettalkoholethoxylaten, Fettalkoholpropoxylaten, Alkylglucosiden, Alkylpolyglucosiden, Oktylphenolethoxylaten, Nonylphenolethoxylaten, Nonoxinolen, Block-Copolymertensiden oder kationischen Tensiden auf Basis von quartären Ammoniumverbindungen, gewählt aus Hexadecyltrimethylammoniumbromid, Distearyldimethylammoniumchlorid oder fluorierten Tensiden oder Silikontensiden verwendet wird. Neben der Verbesserung der Verspinnbarkeit durch Zusatz der spezifisch genannten Tenside zu der Polymermischung gelingt es weiterhin, die Empfindlichkeit eines damit ausgebildeten Sensors zu erhöhen, indem die Porosität der Fasern weiter erhöht wird und somit das Oberflächen-zu-Volumen-Verhältnis weiter verbessert wird, was neben der Verbesserung der Messintensität auch die Ansprechgeschwindigkeit weiter erhöht. Mit derartigen Sensoren ist es beispielsweise möglich, Konzentrationen von 0 bis 100 % von Substanzen, wie O₂, CO₂, Lactat und verschiedenen Ionen sowohl in der Gas- als auch in der Flüssigkeitsphase von Proben sicher und zuverlässig zu messen.

Besonders schnell ansprechende Sensoren werden gemäß einer Weiterbildung der Erfindung dadurch erzielt, dass das Tensid in einer Menge von 0,001 mg/ml bis 10 mg/ml in der zu verspinnenden Polymerlösung enthalten ist. Höhere Mengen an Tensid senken die Oberflächenspannung zu stark ab und es können homogene Fasern nicht mehr erhalten werden. Zu geringe Mengen an Tensid zeigen keine ausreichende Wirkung. Besonders hohe Porenzahlen in den Nanofasern können weiterhin dadurch erreicht werden, dass die Ausgangsmaterialien für die Nanofasern in einem niedrig siedenden, organischen Lösungsmittel, wie C₄ - C₆ Ketone gelöst werden und während und nach der Herstellung der Fasern dafür Sorge getragen wird, dass das Lösungsmittel rasch und vollständig aus den gebildeten Fasern entfernt wird, wodurch die Porenzahl der Fasern weiter erhöht werden kann und es zu keiner Absenkung der Aktivität des Farbstoffindikators durch im Polymer zurückbleibende Lösungsmittelmoleküle kommt.

Insbesondere bevorzugte Ergebnisse werden gemäß der Erfindung dadurch erzielt, dass als Tensid Hexadecyltrimethylammoniumbromid verwendet wird. Durch Zugabe eines derartigen Tensids kann die Empfindlichkeit des Sensors weiter erhöht werden, ohne die Ansprechgeschwindigkeit herabzusetzen.

Besonders gleichmäßige Nanofasern, welche insbesondere reproduzierbare Messungen ermöglichen, werden gemäß der Erfindung dadurch erzielt, dass Polymere auf Polysaccharid-Basis, wie Zellulose, Zelluloseacetat (CA), Hydroxyethylzellulose, Chitosan, Dibutyrylchitin, Carboxymethylzellulose (CMC), Ethylzellulose, Trimethylsilylzellulose (TMSC), Kollagen, Dextran, Gelatine, Gluten, Hyaluronsäure; Ethyl-Vinylalkohol-Copolymer (EVOH), Poly(vinylbutyral) (PVB), Polyamide (PA), Polyacrylsäure (PAA), Polyacrylnitril (PAN), Polycaprolacton (PCL), Polyethyleoxid (PEO), Polyoxymethylen (POM), Polyester, gewählt aus Polyethylen- und butylenterephalat (PET, PBT), Polybutylensuccinat (PBS), Polytrimethylenterephthalat; Polylactide (PLA) und Polyglycolide und deren Copolymere (PLGA), Polyacrylate, gewählt aus Polymethylmethacrylat (PMMA),Polyacrylamide, Polyhydroxyethylacrylat (poly-HEMA), Polyglykole, Polysulfon (PSU), Polyetherimid (PEI), Polythiophene, Polyanilin (PANi), Polysilane, Polyimide, Polypyrrol, Etyl-Vinyl-Acetat Copolymere (EVAc), Polystyrol (PS), Polyurethane (PU), Polyvinylalkohol (PVOH), Polyvinylchlorid (PVC), Polyvinylidenfluorid (PVDF) sowie fluorierte Copolymere, Polyamine, gewählt aus Polyvinylpyrrolidon (PVP), Polycarbonate (PC), Polyvinylcarbazol oder Mischungen aus denselben umfasst sind. Von diesen Polymeren sind Einzelne sowohl in gängigen homogene Schichten aufweisenden Sensoren als auch in Sensoren, welche Nanofasern enthalten, bereits im Einsatz, wobei jedoch es sich überraschenderweise gezeigt hat, dass durch eine Kombination der bevorzugten Lumineszenzfarbstoffe mit den bekannten Polymeren eine deutliche Erhöhung der Ansprechgeschwindigkeit des Sensors erzielt werden kann, so dass mit derartigen Polymeren ausgebildete Sensoren beispielsweise in kardiopulmonaren Belastungstests, der Spiroergometrie, der Beatmungsüberwachung, in der Klinik- und Notfallsmedizin sowie auch in der Stoffwechseldiagnostik und der Lungenfunktionsüberprüfung, wie beispielsweise in der Sportmedizin ihre Anwendung finden können. Insbesondere wenn der Sensor eine homogene Verteilung der Lumineszenzmaterialien in den Polymeren aufweist, d.h. keine Agglomerate der Farbstoffmoleküle gebildet werden, gelingt es, besonders rasche Ansprechzeiten zu erreichen.

Um den Sensor gegen äußere Einflüsse im Wesentlichen unempfindlich zu machen und gleichzeitig jedoch die Ansprechgeschwindigkeit desselben nicht abzusenken, ist gemäß einer Weiterbildung der Erfindung der Sensor so ausgebildet, dass die Sensorschicht mit einer permeablen Deckschicht verwendet wird.

Besonders vorteilhafte Ergebnisse, insbesondere keinerlei Einschränkungen in Bezug auf die Ansprechgeschwindigkeit und die Ansprechintensität werden gemäß der vorliegenden Erfindung dadurch erzielt, dass die permeable Deckschicht aus porösen Membranen, wie Polytetrafluorethylen, Polyvinylidenfluorid, Zelluloseacetat, Zellulosenitrat oder einem Polyamid mit einer Porengröße zwischen 0,1 µm und 2000 µm gebildet ist.

Eine weitere Möglichkeit, um die verwendeten Sensoren gegenüber äußeren Einflüssen im Wesentlichen unempfindlich zu machen und gleichzeitig jedoch die Ansprechgeschwindigkeit desselben nicht abzusenken, ist gemäß einer Weiterbildung der Erfindung der Sensor so ausgebildet, dass die Sensorschicht aus mit der permeablen Deckschicht verbundenen Nanofasern gebildet ist, wobei weiterhin, insbesondere keinerlei Einschränkungen in Bezug auf die Ansprechgeschwindigkeit und die Ansprechintensität werden gemäß der vorliegenden Erfindung dadurch erzielt werden, dass die permeable Deckschicht aus porösen Membranen, wie Polytetrafluorethylen, Polyvinylidenfluorid, Zelluloseacetat, Zellulosenitrat oder Polyamid mit einer Porengröße zwischen 0,1 µm und 2000 µm gebildet ist.

Die Kombination aus permeabler Deckschicht und Sensorschicht kann weiters in beliebiger Weise mit Sensoren oder Sensorsubstraten assembliert werden.

Unter der Vielzahl von herstellbaren Sensoren hat sich die Verwendung als Sauerstoffsensor insbesondere ein opto-chemischer Sensor bewährt, dessen Sensorschicht aus einem mit Pt(II)Tetra(pentafluorophenyl)porphyrin (PtTFPP) dotiertem und mit Hexadecyltrimethylammoniumbromid versetztem Polymer gebildet ist. Mit einem derartigen Sensor gelingt es, einen miniaturisierbaren Sauerstoffsensor herzustellen, welcher Ansprechzeiten von weit unter 1 s aufweist und welcher beispielsweise in der Intensivmedizin, Anästhesie, Lungendiagnostik und Sportmedizin eingesetzt werden kann. Ein derartiger Sensor kann beispielsweise für Messungen der Atemluft direkt in dem Strom der Ausatemluft in der Nähe der Nase angeordnet werden, und indem, wie dies einer Weiterbildung der Erfindung entspricht, der Sensor in einem in einer Montagekappe angeordneten Klemm- bzw. Halteelement angeordnet ist, kann der Sensor einfach gehalten werden und beispielsweise über Glasfasern mit einer Auswerteelektronik verbunden werden, welche beispielsweise ebenfalls miniaturisiert an einem Gürtel oder einer Hosentasche des Benützers getragen werden kann, so dass mit einem derartigen Minisensor eine Vielzahl von Messungen, wie Atemluft in Echtzeit durchgeführt werden können.

Indem weiterhin die Deckschicht im Bereich der Klemm- und Halteelemente mit dem Träger verklebt ausgebildet ist, gelingt es auch, den Sensor vor mechanischen Einflüssen zu schützen, so dass ein verlängerter und insbesondere wiederholter Einsatz desselben ermöglicht ist.

Aufgrund der extrem kurzen Ansprechzeiten des opto-chemischen Sensors und insbesondere aufgrund der Miniaturisierung desselben ist gemäß einer Weiterbildung der Sensor so gebildet, dass die Sensorschicht aus zwei übereinander gelagerten Schichten mit voneinander verschiedenen Lumineszenzfarbstoffen dotierten Nanofasern gebildet ist. Indem die Sensorschicht aus zwei übereinander gelagerten Schichten mit voneinander verschiedenen Lumineszenzfarbstoffen dotierten Nanofasern gebildet ist, gelingt es gleichzeitig, entweder zwei Eigenschaften eines Stoffs, wie beispielsweise die Sauerstoffkonzentration und den pH-Wert oder zwei voneinander verschiedene Analyten zu messen. Eine derartige Anordnung ist aufgrund der großen Porosität der Schichten, welche schnelle Diffusionsgeschwindigkeiten der jeweiligen Analyten bis in tiefe Schichten des Sensors ermöglichen, präzise und weist für beide Messgrößen Ansprechzeiten unter 1 s auf.

Eine derartige Kombination von Farbstoffen kann gemäß einer Weiterbildung der Erfindung auch in nur einer Nanofaserschicht realisiert werden, bei welcher die Sensorschicht aus zwei voneinander verschiedenen Lumineszenzfarbstoffen enthaltenen Nanofasern gebildet ist. Derartige Sensoren können für die Messung von Analyten basierend auf den Prinzipien von DLR (Dual Luminophor Referencing, RET (Resonance Energy Transfer) sowie light Harvesting eingesetzt werden.

Um insbesondere sicherzustellen, dass sämtliche Analyten auch tiefer gelegene Nanofaserschichten erreichen, ist gemäß einer Weiterbildung der Erfindung der opto-chemische Sensor so ausgebildet, dass übereinander angeordnete, mit Lumineszenzfarbstoffen dotierte Nanofaserschichten voneinander verschiedene Schichtstärken aufweisen.

Reproduzierbare und sichere Messergebnisse werden gemäß der vorliegenden Erfindung dadurch erzielt, dass die Schichtstärke jeder mit einem Lumineszenzfarbstoff dotierten, aus Nanofasern bestehenden Schicht mindestens die einfache Dicke einer Nanofaser, d.h. 50 nm bis 1000 nm beträgt.

Die Erfindung wird nachfolgend anhand von Ausführungsbeispielen näher erläutert.

### Beispiel 1

### Herstellung eines Sauerstoffsensors

Eine 15 gew.-%ige Polystyrollösung wird mit 0,1 Gew.-% Pt-Tetra(pentafluorphenyl)-porphyrin (PtPFTT) und 0,008 Gew.-% Hexadecyltrimethylammoniumbromid in 2-Butanon gelöst und mit einer Spannung von 12 kV und 12 cm Abstand zwischen einer Metallnadel, aus welcher die Lösung ausgepresst wird, und einer Gegenelektrode zu einer Faserschicht mit einer Dicke von 20 µm auf einer Polyethylenterephthalat-(PET)-Folie versponnen. Die Nanofaserschicht wird mit einer Polyvinylidendifluoridmembran mit 0,22 nm Porengröße abgedeckt und mit der PET-Seite auf einem Glasstab montiert und in einer Klemmeinrichtung gehalten, um ein Ablösen der Polyvinylidendifluoridschicht hintanzuhalten. Über den Glasstab wird der so gebildete Sensor mit Licht angeregt und das emittierte Licht zu einer Photodiode abgeleitet.

In einem Einsatz zur Messung der Sauerstoffkonzentration von Atemluft zeigte der Sensor Ansprechzeiten t₉₅, dies ist die Zeit, bis 95 % des konstanten Endsignals erreicht werden, von 80 ms. Zum Vergleich zeigt ein Sensor, welcher eine homogene aus denselben Materialien ausgebildete Sensorschicht aufweist, eine Ansprechdauer von 5 s.

### Beispiel 2

### Herstellung eines Sauerstoffsensors

Eine 15 gew.-%ige Polystyrollösung wird mit 0,09 Gew.-% Pd-meso-Tetra(pentafluorphenyl)porphyrin und 0,1 Gew.-% Distearyldimethylammoniumchlorid in 2-Butanon gelöst und gemäß dem Verfahren von Beispiel 1 verarbeitet.

In einem Einsatz zur Messung der Sauerstoffkonzentration von vakuumverpackten Lebensmitteln zeigte der Sensor Ansprechzeiten t₉₅ von 75 ms. Zum Vergleich zeigt ein Sensor, welcher eine homogene aus denselben Materialien ausgebildete Sensorschicht aufweist, eine Ansprechdauer von 5,10 s.

### Beispiel 3

### Herstellung eines Sauerstoffsensors

Eine 15 gew.-%ige Polymethylmethacrylat (PMMA) wird mit 0,1 Gew.-% Ru(II)-Tris(4,7-diphenyl-1,10-phenanthrolin) und 0,007 Gew.-% Dodecylsulfonat in 2-Butanon gelöst und gemäß dem Verfahren von Beispiel 1 verarbeitet.

In einem Einsatz zur Messung der Sauerstoffkonzentration eines Ausdauersportlers zeigte der Sensor Ansprechzeiten t₉₅ von 85 ms. Zum Vergleich zeigt ein Sensor, welcher eine homogene aus denselben Materialien ausgebildete Sensorschicht aufweist, eine Ansprechdauer von 5 s.

### Beispiel 4

### Herstellung eines Sauerstoffsensors

Eine 15 gew.-%ige Carboxymethylzelluloselösung wird mit 0,12 Gew.-% Tris(2,2-bipyridin)ruthenium(II)chlorid Hexahydrat in 2-Pentanon gelöst und gemäß dem Verfahren von Beispiel 1 verarbeitet.

In einem Einsatz zur Messung der Sauerstoffkonzentration eines an COPD leidenden Patienten zeigte der Sensor Ansprechzeiten t₉₅ von 80 ms. Zum Vergleich zeigt ein Sensor, welcher eine homogene aus denselben Materialien ausgebildete Sensorschicht aufweist, eine Ansprechdauer von 5,2 s.

### Beispiel 5

### Herstellung eines CO₂-Sensors

Eine 15 gew.-%ige Polyvinylbutyrallösung wird in einer Toluol/Ethanol-Mischung (80 % / 20 %) mit 0,15 % Cresolrot und Thymolblau und mit 0,01 % Tetraoctylammoniumhydroxid als Ionenpaarbildner und 0,005 Gew.-% Triton X-100 gelöst und gemäß dem Verfahren von Beispiel 1 verarbeitet.

Im Einsatz zur Messung des CO₂-Gehalts aus Atemluft eines Sportlers zeigte ein derartiger Sensor eine Ansprechzeit t₉₅ von 90 ms. Im Vergleich zeigt ein herkömmlicher CO₂-Sensor eine Ansprechzeit von 5 s.

### Beispiel 6

### Herstellung eines CO₂-Sensors

Eine 12 gew.-%ige Polyvinylpyrolidonlösung wird in einer Toluol/Ethanol-Mischung (80 % / 20 %) mit 0,1 % 8-Hycroxypyren-1,3,6-trisulfonsäure (HPTS) und mit 0,01 % Tetraoctylammoniumhydroxid als Ionenparabildner gelöst und gemäß dem Verfahren von Beispiel 1 verarbeitet.

Im Einsatz zur Messung des CO₂-Gehalts aus Atemluft eines Sportlers zeigte ein derartiger Sensor eine Ansprechzeit t₉₅ von 85 ms. Im Vergleich zeigt ein herkömmlicher CO₂-Sensor eine Ansprechzeit von 5,5 s.

### Beispiel 7

### Herstellung eines CO₂-Sensors

Eine 12 gew.-%ige Ethylzelluloselösung wird in einer Toluol/Ethanol-Mischung (80 % / 20 %) mit 0,1 % Xylenolblau (XB) und mit 0,009 % Dodecylsulfat gelöst und gemäß dem Verfahren von Beispiel 1 verarbeitet.

Im Einsatz zur Messung des CO₂-Gehalts aus Atemluft eines an COPD leidenden Patienten zeigte ein derartiger Sensor eine Ansprechzeit t₉₅ von 80 ms. Im Vergleich zeigt ein herkömmlicher CO₂-Sensor eine Ansprechzeit von 5 s.

### Beispiel 8

### Herstellung eines CO₂-Sensors

Eine 12 gew.-%ige Polyvinylalkohollösung wird in einer Toluol/Ethanol-Mischung (80 % / 20 %) mit 0,1 % m-Cresolrot (mCP) und mit 0,008 % Natriumlaurylsulfat gelöst und gemäß dem Verfahren von Beispiel 1 verarbeitet.

In einem Einsatz zur Messung des CO₂-Gehalts aus Atemluft eines anastethischen Patienten zeigte ein derartiger Sensor eine Ansprechzeit t₉₅ von 75 ms. Im Vergleich zeigt ein herkömmlicher CO₂-Sensor eine Ansprechzeit von 5,3 s.

### Beispiel 9

### Herstellung eines pH-Sensors

Eine 10 gew.-%ige Polyvinylalkohollösung wird in einer Ethanol/H₂O-Mischung (50 % / 50 %) mit 0,2 % Phenolrot und mit 0,005 % Dodecylsulfat gelöst und gemäß dem Verfahren von Beispiel 1 verarbeitet.

In einem Einsatz zur Messung des Blut-pH-Werts zeigte ein derartiger Sensor eine Ansprechzeit t₉₅ von knapp 1 s. Im Vergleich zeigt ein herkömmlicher pH-Sensor eine Ansprechzeit von 4,9 s.

### Beispiel 10

### Herstellung eines pH-Sensors

Eine 10 gew.-%ige Polystyrollösung wird in einer Ethanol/H₂O-Mischung (50 % / 50 %) mit 0,25 % 2',7'-Dichlorfluorescein und mit 0,005 % Distearyldimethylammoniumchlorid gelöst und gemäß dem Verfahren von Beispiel 1 verarbeitet.

In einem Einsatz zur Messung des Blut-pH-Werts zeigte ein derartiger Sensor eine Ansprechzeit t₉₅ von knapp 80 ms. Im Vergleich zeigt ein herkömmlicher pH-Sensor eine Ansprechzeit von 5,2 s.

### Beispiel 11

### Herstellung eines pH-Sensors

Eine 10 gew.-%ige Polyamidlösung wird in einer Ethanol/Methanol-Mischung (50 % / 50 %) mit 0,22 % Carboxynaphtofluorescein und mit 0,005 % Dodecylsulfat gelöst und gemäß dem Verfahren von Beispiel 1 verarbeitet.

Im Einsatz zur Messung des pH-Werts, welcher über das CO₂-Kohlensäure-Gleichgewicht zur Ermittlung des CO₂-Partialdrucks der Ausatemluft eines an Asthma erkrankten Patienten eingesetzt wurde, zeigte ein derartiger Sensor eine Ansprechzeit t₉₅ von knapp 80 ms. Im Vergleich zeigt ein herkömmlicher pH-Sensor eine Ansprechzeit von 5,6 s.

### Beispiel 12

### Herstellung eines CO₂-Sensors basierend auf RET (Resonance Energy Transfer)

Eine 15 gew.-%ige Ethylzelluloselösung wird in einer Toluo/Ethanol-Mischung (80 % / 20 %) mit 0,19 % Phenolrot und 5 % Ruthenium (Diphenylphenanthrolin) als Nanobeads in PAN und mit 0,01 % Tetraoctylammoniumhydroxid als Ionenpaarbinder gelöst und gemäß dem Verfahren von Beispiel 1 verarbeitet.

Im Einsatz zur Messung des CO₂ Gehalts aus Atemluft eines Sportlers zeigte ein derartiger Sensor eine Ansprechzeit t₉₅ von 90 ms. Im Vergleich zeigt ein herkömmlicher CO₂-Sensor eine Ansprechzeit von 5 s.

### Beispiel 13

### Herstellung eines CO₂-Sensors basierend auf DLR (Dual Luminophor Referencing)

Eine 30 gew.-%ige Eudragit RL 100-Lösung wird in Ethanol/Waser 9/1 % mit 0,1 % HPTS und 0,5 % Ruthenium (Diphenylphenanthrolin) als Nanobeads in PAN und mit 0,01 % Tetraoctylammoniumhydroxid als Ionenpaarbildner gelöst und gemäß dem Verfahren von Beispiel 1 verarbeitet.

Im Einsatz zur Messung des CO₂-Gehalts aus Atemluft eines Sportlers zeigte ein derartiger Sensor eine Ansprechzeit t₉₅ von 90 ms. Im Vergleich zeigt ein herkömmlicher CO₂-Sensor eine Ansprechzeit von 5 s.

### Beispiel 14

### Herstellung eine NH₂-Sensors basierend auf einer Light Harvesting-Kaskade

Eine 15 gew.-%ige PMMA-Lösung wird in Aceton mit 1 % Coumarin 545T, 2 mmol/kg Eosin gemäß dem Verfahren von Beispiel 1 verarbeitet.

Im Einsatz zur Messung des HN₃-Gehalts aus Atemluft eines Sportlers zeigte ein derartiger Sensor eine Ansprechzeit von 90 ms. Im Vergleich zeigt ein herkömmlicher NH₃-Sensor eine Ansprechzeit von 5 s.

## Patentansprüche

1. Opto-chemischer Sensor, umfassend eine auf einen Träger aufgebrachte aus mit einem Lumineszenzfarbstoff dotierten, gesponnenen Nanofasern gebildete Sensorschicht, deren Emissionsvermögen nach Anregung mit elektromagnetischer Strahlung durch nachzuweisende Substanzen, gewählt aus O₂, CO₂, Lactat, Glucose, NH₃, Stickoxide und Ionen bzw. zu ermittelnde Messgrößen, gewählt aus dem pH-Wert, der Feuchte und der Temperatur in einer Gas- oder Flüssigphase, veränderbar ist,
wobei die Sensorschicht aus Nanofasern mit einem Durchmesser zwischen 50 nm und 1000 nm gebildet ist,
wobei die Nanofasern ein Polymer umfassen, ausgewählt aus der Gruppe, bestehend aus Polymere auf Polysaccharid-Basis, gewählt aus Zellulose, Zelluloseacetat (CA), Chitosan, Carboxymethylzellulose (CMC), Ethylzellulose, Trimethylsilylzellulose (TMSC) oder Hyaluronsäure; Ethyl-Vinylalkohol-Copolymer (EVOH), Poly(vinylbutyral) (PVB), Polyamide (PA), Polyacrylsäure (PAA), Polyacrylnitril (PAN), Polyethylenoxid (PEO), Polyoxymethylen (POM), Polyester, gewählt aus Polyethylen- und butylenterephalat (PET, PBT) oder Polytrimethylenterephthalat; Polylactide (PLA) und Polylactid-Polyglyolid-Copolymere (PLGA), Polyacrylate, gewählt aus Polymethylmethacrylat (PMMA) oder Polyacrylamide, Polyglykole, Polysulfon (PSU), Polyetherimid (PEI), Polythiophene, Polyanilin (PANi), Polysilane, Polyimide, Ethyl-Vinyl-Acetat Copolymere (EVAc), Polystyrol (PS), Polyurethane (PU), Polyvinylalkohol (PVOH), Polyvinylchlorid (PVC), Polyvinylidenfluorid (PVDF) sowie flourierte Copolymere, Polyamine, Polyvinylpyrrolidon (PVP), Polycarbonate (PC) oder Mischungen aus denselben,
wobei die Nanofasern mit wenigstens einem Lumineszenzfarbstoff, gewählt aus der Gruppe der Metallporphyrine, Benzoporphyrine, Napthoporphyrine, Phthallocyanine, Perylendiimine, Pyrene; Xanthenfarbstoffe, Azofarbstoffe, Bodipyfarbstoffe, Bipyridine, Phenantroline, Coumarine und Acetylacetonate von Ruthenium und Iridium; Acridinfarbstoffe, Oxazinfarbstoffe, Coumarine, Azaannulene, Squarine, 8-Hydroxychinoline, lumineszenten Nanopartikel, gewählt aus Quantenpunkten oder Nanokristallen; Carbostyryle, Terbiumkomplexe, anorganischen Phosphore oder mit lonophoren verbundene oder derivatisierte Farbstoffe der vorhin genannten Klassen dotiert sind, und
wobei die Nanofasern weiterhin ein anionisches, kationisches oder nicht ionisches Tensid enthalten.

2. Opto-chemischer Sensor nach Anspruch 1, **dadurch gekennzeichnet, dass** der wenigstens eine Lumineszenzfarbstoff gewählt ist aus: Pd(II)-Octaethylporphyrin, Pt(II)-Octaethylporphyrin, Pd(II)-Tetraphenylporphyrin, Pt(II)-Tetraphenylporphyrin, Pd(II)-meso-Tetraphenyl-tetrabenzoporphyrin, Pt(II)-meso-Tetraphenyl-tetrabenzoporphyrin, Pd(II)-Octaethylporphyrinketon, Pt(II)-Octaethylporphyrinketon, Pd(II)-meso-Tetra(pentafluorphenyl) porphyrin, Pt(II)-meso-Tetra(pentafluorphenyl)porphyrin, Ru(II)-Tris(4,7-diphenyl-1,10-phenanthrolin), Ru(II)-Tris(1,10-phenanthrolin), Tris(2,2-bipyridine)ruthenium(II)chlorid Hexahydrat [Ru(bpy)3], Fluorescein, Iridium(III)((N-methyl-benzoimidazol-2-yl)-7-(diethylamino)-coumarin))2(acetylacetonat), Lumogenrot, Macrolex Fluoreszenz gelb, Rhodamin B, Rhodamin 6G, Texasrot, Thymolblau (TB), Xylenolblau (XB), Cresolrot (CR), Bromcresolgrün (BG), Bromthymolblau (BTB), 4-Nitrophenol (NP), Alizarin, Phenolphtalein (PP), o-Cresolphtalein (oCP), Calmagit (CG), Bromxylenolblau (BXB), Methylrot (MR), Phenolrot (PR), Neutralrot (NR), Nitrazingelb (NY), 3,4,5,6 Tetrabromphenolsulfonphtalein, Kongorot (CR), 5(6)-Carboxy-fluorescein, Carboxynaphtofluorescein, 8-Hydroxypyren-1,3,6-trisulfonsäure (HPTS), Seminaphthorhodafluors (SNARF Farbstoffe) Seminaphtofluorescein (SNAFL Farbstoffe) oder aus mit Kronenether verbundene oder derivatisierte vorhin genannte Farbstoffe.

3. Opto-chemischer Sensor nach Anspruch 1 oder 2, **dadurch gekennzeichnet, dass** das Tensid aus der Gruppe bestehend aus anionischen Tensiden auf Basis von Carboxylaten, Sulfonaten und Sulfaten, wie Alkylcarboxylate, Alkylbenzolsulfonate, Natriumdodecylbenzolsulfonat, sekundäre Alkansulfonate, Sulfate wie Natriumlaurylsulfat, Dodecylsulfat, nichtionischen Tensiden auf Basis von Fettalkoholethoxylaten, Fettalkoholpropoxylaten, Alkylglucosiden, Alkylpolyglucosiden, Oktylphenolethoxylaten, Nonylphenolethoxylaten, Nonoxinolen, Block-Copolymertensiden oder kationischen Tensiden auf Basis von quartären Ammoniumverbindungen, wie Hexadecyltrimethylammoniumbromid, Distearyldimethylammoniumchlorid oder fluorierte Tenside oder Silikontenside gewählt ist.

4. Opto-chemischer Sensor nach einem der Ansprüche 1 bis 3, **dadurch gekennzeichnet, dass** als Tensid Hexadecyltrimethylammoniumbromid eingesetzt ist.

5. Opto-chemischer Sensor nach einem der Ansprüche 1 bis 4, **dadurch gekennzeichnet, dass** die Sensorschicht mit einer permeablen Deckschicht abgedeckt ist.

6. Opto-chemischer Sensor nach einem der Ansprüche 1 bis 4, **dadurch gekennzeichnet, dass** die Sensorschicht aus mit der permeablen Deckschicht verbundenen Nanofasern gebildet ist.

7. Opto-chemischer Sensor nach Anspruch 5 oder 6, **dadurch gekennzeichnet, dass** die permeable Deckschicht aus porösen Membranen, Polytetrafluorethylen, Polyvinylidenfluorid TF, Zelluloseacetat, Zellulosenitrat oder Polyamid mit einer Porengröße zwischen 0,1 µm und 2000 µm gebildet ist.

8. Opto-chemischer Sensor nach einem der Ansprüche 1 bis 7, **dadurch gekennzeichnet, dass** die Sensorschicht aus mit Pt(II)-Tetra(pentafluorphenyl)porphyrin (PtPFTT) dotiertem und mit Hexadecyltrimethylammoniumbromid versetztem Polymer gebildet ist.

9. Opto-chemischer Sensor nach einem der Ansprüche 1 bis 8, **dadurch gekennzeichnet, dass** die Sensorschicht aus zwei übereinander gelagerten Schichten mit voneinander verschiedenen Lumineszenzfarbstoffen dotierten Nanofasern gebildet ist.

10. Opto-chemischer Sensor nach einem der Ansprüche 1 bis 8, **dadurch gekennzeichnet, dass** die Sensorschicht aus zwei voneinander verschiedene Lumineszenzfarbstoffe enthaltenen Nanofasern gebildet ist.

11. Opto-chemischer Sensor nach einem der Ansprüche 1 bis 10, **dadurch gekennzeichnet, dass** die Schichtstärke jeder mit einem Lumineszenzfarbstoff dotierten, aus Nanofasern bestehenden Schicht mindestens die einfache Dicke einer Nanofaser, also 50 nm bis 1000 nm beträgt.

12. Opto-chemischer Sensor nach einem der Ansprüche 1 bis 11, **dadurch gekennzeichnet, dass** übereinander angeordnete, mit Lumineszenzfarbstoffen dotierte Nanofaserschichten voneinander verschiedene Schichtstärken aufweisen.

13. Opto-chemischer Sensor nach einem der Ansprüche 1 bis 12, **dadurch gekennzeichnet, dass** der Sensor in einem in einer Montagekappe angeordneten Klemm- bzw. Halteelement angeordnet ist.

14. Opto-chemischer Sensor nach einem der Ansprüche 1 bis 13, **dadurch gekennzeichnet, dass** die Deckschicht im Bereich des Klemm- bzw. Halteelements mit dem Träger verklebt ausgebildet ist.

15. Verwendung einer auf einen Träger aufgebrachten aus mit einem Lumineszenzfarbstoff dotierten, gesponnenen Nanofasern gebildeten Sensorschicht, deren Emissionsvermögen nach Anregung mit elektromagnetischer Strahlung durch nachzuweisende Substanzen veränderbar ist, als opto-chemischer Sensor für O₂, CO₂, Lactat, Glucose, NH₃, Stickoxide und Ionen bzw. zu ermittelnde Messgrößen, gewählt aus dem pH-Wert, der Feuchte und der Temperatur in einer Gas- oder Flüssigphase, mit einer Ansprechzeit t₉₅ von unter 1 s,
wobei die Sensorschicht aus Nanofasern mit einem Durchmesser zwischen 50 nm und 1000 nm gebildet ist,
wobei die Nanofasern ein Polymer umfassen, ausgewählt aus der Gruppe, bestehend aus Polymere auf Polysaccharid-Basis, gewählt aus Zellulose, Zelluloseacetat (CA), Chitosan, Carboxymethylzellulose (CMC), Ethylzellulose, Trimethylsilylzellulose (TMSC) oder Hyaluronsäure; Ethyl-Vinylalkohol-Copolymer (EVOH), Poly(vinylbutyral) (PVB), Polyamide (PA), Polyacrylsäure (PAA), Polyacrylnitril (PAN), Polyethylenoxid (PEO), Polyoxymethylen (POM), Polyester, gewählt aus Polyethylen- und butylenterephalat (PET, PBT) oder Polytrimethylenterephthalat; Polylactide (PLA) und Polylactid-Polyglyolid-Copolymere (PLGA), Polyacrylate, gewählt aus Polymethylmethacrylat (PMMA) oder Polyacrylamide, Polyglykole, Polysulfon (PSU), Polyetherimid (PEI), Polythiophene, Polyanilin (PANi), Polysilane, Polyimide, Ethyl-Vinyl-Acetat Copolymere (EVAc), Polystyrol (PS), Polyurethane (PU), Polyvinylalkohol (PVOH), Polyvinylchlorid (PVC), Polyvinylidenfluorid (PVDF) sowie flourierte Copolymere, Polyamine, Polyvinylpyrrolidon (PVP), Polycarbonate (PC) oder Mischungen aus denselben,
wobei die Nanofasern mit wenigstens einem Lumineszenzfarbstoff, gewählt aus der Gruppe der Metallporphyrine, Benzoporphyrine, Napthoporphyrine, Phthallocyanine, Perylendiimine, Pyrene; Xanthenfarbstoffe, Azofarbstoffe, Bodipyfarbstoffe, Bipyridine, Phenantroline, Coumarine und Acetylacetonate von Ruthenium und Iridium; Acridinfarbstoffe, Oxazinfarbstoffe, Coumarine, Azaannulene, Squarine, 8-Hydroxychinoline, lumineszenten Nanopartikel, gewählt aus Quantenpunkten oder Nanokristallen; Carbostyryle, Terbiumkomplexe, anorganischen Phosphore oder mit lonophoren verbundene oder derivatisierte Farbstoffe der vorhin genannten Klassen dotiert sind, und
wobei die Nanofasern weiterhin ein anionisches, kationisches oder nicht ionisches Tensid enthalten.

## Claims

1. An opto-chemical sensor comprising a sensor layer arranged on a carrier and formed from spun nanofibers doped with a luminescent dye, wherein the sensor layer has an emissivity after excitation with electromagnetic radiation that can be changed by substances to be detected selected from O₂, CO₂, lactate, glucose, NH₃, nitrogen oxides and ions or measured variables to be determined selected from the pH-value, the moisture and the temperature in a gas or liquid phase,
wherein the sensor layer is formed from nanofibers having a diameter between 50 nm and 1000 nm,
wherein the nanofibers comprise a polymer selected from the group consisting of polysaccharide-based polymers selected from cellulose, cellulose acetate (CA), chitosan, carboxymethyl cellulose (CMC), ethyl cellulose, trimethylsilyl cellulose (TMSC) or hyaluronic acid; ethylene vinyl alcohol copolymer (EVOH), poly (vinyl butyral) (PVB), polyamide (PA), polyacrylic acid (PAA), polyacrylonitrile (PAN), polyethyleneoxide (PEO), polyoxymethylene (POM), polyester selected from polyethylene and polybutylene terephthalate (PET, PBT) or polytrimethylene terephthalate; polylactide (PLA) and polylactide polyglycolide copolymers (PLGA), polyacrylates selected from poly(methyl methacrylate) (PMMA) or polyacrylamides, polyglycols, polysulfone (PSU), polyetherimide (PEI), polythiophene, polyaniline (PANi), polysilanes, polyimides, ethylene-vinyl acetate copolymers (EVAc), polystyrol (PS), polyurethanes (PU), polyvinyl alcohol (PVOH), polyvinyl chloride (PVC), polyvinylidene fluoride (PVDF) as well as fluorinated copolymers, polyamines, polyvinylpyrrolidone (PVP), polycarbonates (PC) or mixtures thereof,
wherein the nanofibers are doped with at least one luminescent dye selected from the group of metal porphyrins, benzoporphyrins, naphthoporphyrins, phthalocyanines, perylene diimines, pyrenes; xanthene dyes, azo dyes, bodipy dyes, bipyridines, phenantrolines, coumarins and acetyl acetonates of ruthenium and iridium; acridine dyes, oxazine dyes, coumarins, azaannulenes, squarines, 8-hydroxyquinolines, luminescent nanoparticles selected from quantum dots or nanocrystals; carbostyryls, terbium complexes, inorganic phosphors or dyes of the aforementioned classes derivatized or bound to ionophores, and
wherein the nanofibers further contain an anionic, cationic or nonionic surfactant.

2. The opto-chemical sensor according to claim 1, **characterized in that** the at least one luminescent dye is selected from Pd(II) octaethylporphyrin, Pt(II) octaethylporphyrin, Pd(II) tetraphenylporphyrin, Pt(II) tetraphenylporphyrin, Pd(II) meso-tetraphenyl-tetrabenzoporphyrin, Pt(II) meso-tetraphenyl-tetrabenzoporphyrin, Pd(II) octaethylporphyrinketone, Pt(II) octaethylporphyrinketone, Pd(II) meso-tetra(pentafluorophenyl) porphyrin, Pt(II) meso-tetra(pentafluorophenyl) porphyrin, Ru(II) tris(4,7-diphenyl-1,10-phenanthrolin), Ru(II) tris(1,10-phenanthrolin), tris(2,2-bipyridine) ruthenium(II) chloride hexahydrate [Ru(bpy)3], fluorescein, iridium(III) ((N-methyl-benzoimidazol-2-yl)-7-(diethylamino)-coumarin))2(acetylacetonate), Lumogen Red, Macrolex Fluorescent Yellow, rhodamine B, rhodamine 6G, Texas Red, thymol blue (TB), xylenol blue (XB), cresol red (CR), bromocresol green (BG), bromothymol blue (BTB), 4-nitrophenol (NP), alizarin, phenolphtalein (PP), o-cresolphtalein (oCP), calmagite (CG), bromoxylenol blue (BXB), methyl red (MR), phenol red (PR), neutral red (NR), nitrazine yellow (NY), 3,4,5,6 tetrabromophenol sulfonephthalein, Congo red (CR), 5(6)-carboxyfluorescein, carboxynaphthofluorescein, 8-hydroxypyrene-1,3,6-trisulfonic acid (HPTS), seminaphtharhodafluors (SNARF dyes), seminaphtafluorescein (SNAFL dyes) or the aforementioned dyes derivatized or bound to crown ethers.

3. The opto-chemical sensor according to claim 1 or 2, **characterized in that** the surfactant is selected from the group consisting of anionic surfactants based on carboxylates, sulfonates and sulfates, such as alkyl carboxylates, alkylbenzene sulfonates, sodium dodecylbenzensulfonate, secondary alkanesulfonates, sulfates, such as sodium lauryl sulfate, dodecyl sulfate, nonionic surfactants based on fatty alcohol ethoxylates, fatty alcohol propoxylates, alkyl glucosides, alkyl polyglucosides, octylphenol ethoxylates, nonylphenol ethoxylates, Nonoxynoles, block copolymer surfactants or cationic surfactants based on quaternary ammonium compounds, such as hexadecyltrimethylammonium bromide, distearyldimethylammonium chloride or fluorinated surfactants or silicone surfactants.

4. The opto-chemical sensor according to any one of claims 1 to 3, **characterized in that** hexadecyltrimethylammonium bromide is used as the surfactant.

5. The opto-chemical sensor according to any one of claims 1 to 4, **characterized in that** the sensor layer is covered with a permeable cover layer.

6. The opto-chemical sensor according to any one of claims 1 to 4, **characterized in that** the sensor layer is formed from nanofibers connected to the permeable cover layer.

7. The opto-chemical sensor according to claim 5 or 6, **characterized in that** the permeable cover layer is formed from porous membranes, polytetrafluoroethylene, polyvinylidene fluoride TF, cellulose acetate, cellulose nitrate or polyamide having a pore size between 0.1 µm and 2000 µm.

8. The opto-chemical sensor according to any one of claims 1 to 7, **characterized in that** the sensor layer is formed from a polymer doped with Pt(II) tetra(pentafluorophenyl) porphyrin (PtPFTT) and mixed with hexadecyltrimethylammonium bromide.

9. The opto-chemical sensor according to any one of claims 1 to 8, **characterized in that** the sensor layer is formed from two superposed layers of nanofibers doped with different luminescent dyes.

10. The opto-chemical sensor according to any one of claims 1 to 8, **characterized in that** the sensor layer is formed from nanofibers containing two different luminescent dyes.

11. The opto-chemical sensor according to any one of claims 1 to 10, **characterized in that** the layer thickness of each layer consisting of nanofibers doped with a luminescent dye is at least the single thickness of a nanofiber, i.e. 50 nm to 1000 nm.

12. The opto-chemical sensor according to any one of claims 1 to 11, **characterized in that** nanofiber layers arranged one above the other and doped with luminescent dyes have different layer thicknesses.

13. The opto-chemical sensor according to any one of claims 1 to 12, **characterized in that** the sensor is arranged in a clamping or supporting element arranged in a mounting cap.

14. The opto-chemical sensor according to any one of claims 1 to 13, **characterized in that** the cover layer is glued with the carrier in the region of the clamping or supporting element.

15. Use of a sensor layer arranged on a carrier and formed from spun nanofibers doped with a luminescent dye, wherein the sensor layer has an emissivity after excitation with electromagnetic radiation that can be changed by substances to be detected, as an opto-chemical sensor for O₂, CO₂, lactate, glucose, NH₃, nitrogen oxides and ions or measured variables to be determined selected from the pH-value, the moisture and the temperature in a gas or liquid phase, the opto-chemical sensor having a response time t₉₅ of less than 1 s,
wherein the sensor layer is formed from nanofibers having a diameter between 50 nm and 1000 nm,
wherein the nanofibers comprise a polymer selected from the group consisting of polysaccharide-based polymers selected from cellulose, cellulose acetate (CA), chitosan, carboxymethyl cellulose (CMC), ethyl cellulose, trimethylsilyl cellulose (TMSC) or hyaluronic acid; ethylene vinyl alcohol copolymer (EVOH), poly (vinyl butyral) (PVB), polyamide (PA), polyacrylic acid (PAA), polyacrylonitrile (PAN), polyethyleneoxide (PEO), polyoxymethylene (POM), polyester selected from polyethylene and polybutylene terephthalate (PET, PBT) or polytrimethylene terephthalate; polylactide (PLA) and polylactide polyglycolide copolymers (PLGA), polyacrylates,selected from poly(methyl methacrylate) (PMMA) or polyacrylamides, polyglycols, polysulfone (PSU), polyetherimide (PEI), polythiophene, polyaniline (PANi), polysilanes, polyimides, ethylene-vinyl acetate copolymers (EVAc), polystyrol (PS), polyurethanes (PU), polyvinyl alcohol (PVOH), polyvinyl chloride (PVC), polyvinylidene fluoride (PVDF) as well as fluorinated copolymers, polyamines, polyvinylpyrrolidone (PVP), polycarbonates (PC) or mixtures thereof,
wherein the nanofibers are doped with at least one luminescent dye selected from the group of metal porphyrins, benzoporphyrins, naphthoporphyrins, phthalocyanines, perylene diimines, pyrenes; xanthene dyes, azo dyes, bodipy dyes, bipyridines, phenantrolines, coumarins and acetyl acetonates of ruthenium and iridium; acridine dyes, oxazine dyes, coumarins, azaannulenes, squarines, 8-hydroxyquinolines, luminescent nanoparticles selected from quantum dots or nanocrystals; carbostyryls, terbium complexes, inorganic phosphors or dyes of the aforementioned classes derivatized or bound to ionophores, and
wherein the nanofibers further contain an anionic, cationic or nonionic surfactant.

## Revendications

1. Capteur opto-chimique, comprenant une couche de capteur appliquée sur un support, formée à partir de nanofibres tissées dopées avec un colorant luminescent, dont l'émissivité peut être modifiée après excitation avec un rayonnement électromagnétique par des substances à détecter choisies parmi O₂, CO₂, lactate, glucose, NH₃, oxydes d'azote et des ions ou des grandeurs de mesure à déterminer, choisies parmi la valeur pH, l'humidité et la température dans une phase gazeuse ou liquide,
dans lequel la couche de capteur est formée à partir de nanofibres avec un diamètre entre 50 nm et 1000 nm,
dans lequel les nanofibres comprennent un polymère, choisi parmi le groupe constitué de polymères à base de polysaccharide, choisis parmi la cellulose, l'acétate de cellulose (CA), le chitosane, la carboxyméthylcellulose (CMC), l'éthylcellulose, la triméthylsilylcellulose (TMSC) ou l'acide hyaluronique ; le copolymère d'éthyle-alcool vinylique (EVOH), le polyvinyle de butyral (PVB), les polyamides (PA), l'acide polyacrylique (PAA), le polyacrylonitrile (PAN), l'oxyde de polyéthylène (PEO), le polyoxyméthylène (POM), le polyester, choisi parmi le téréphtalate de polyéthylène et de butylène (PET, PBT) ou le téréphtalate de polytriméthylène ; les polylactides (PLA) et les copolymères de polylactide-polyglyolide (PLGA), les polyacrylates, choisis parmi le méthacrylate de polyméthyle (PMMA) ou les polyacrylamides, les polyglycols, le polysulfone (PSU), le polyétherimide (PEI), le polythiophène, la polyaniline (PANi), les polysilanes, les polyimides, les copolymères d'acétate d'éthyle-vinyle (EVAc), le polystyrène (PS), les polyuréthanes (PU), l'alcool polyvinylique (PVOH), le chlorure de polyvinyle (PVC), le fluorure de polyvinylidène (PVDF) ainsi que les copolymères fluorés, la polyamine, la polyvinylpyrrolidone (PVP), les polycarbonates (PC) ou des mélanges de ceux-ci,
dans lequel les nanofibres sont dopées avec au moins un colorant luminescent choisi parmi le groupe comprenant la métallo-porphyrine, la benzoporphyrine, la naphtoporphyrine, la phtallocyanine, le pérylènediimine, le pyrène ; les colorants du xanthène, les colorants azoïques, les colorants bodipy, la bipyridine, la phénantroline, la coumarine et l'acétylacétonate de ruthénium et d'iridium ; les colorants d'acridine, les colorants d'oxazine, la coumarine, l'aza-annulène, la squarine, la 8-hydroxyquinoléine, les nanoparticules luminescentes, choisies parmi des points quantiques ou des nanocristaux ; le carbostyryle, les complexes de terbium, des phosphores inorganiques ou les colorants liés à des ionophores ou dérivés des classes mentionnées plus haut, et
dans lequel les nanofibres contiennent par ailleurs un agent tensioactif anionique, cationique ou non ionique.

2. Capteur opto-chimique selon la revendication 1, **caractérisé en ce que** l'au moins un colorant luminescent est choisi parmi : Pd(II)-octaéthylporphyrine, Pt(II)-octaéthylporphyrine, Pd(II)-tétraphénylporphyrine, Pt(II)-tétraphénylporphyrine, Pd(II)-mésotétraphényl-tétra-benzoporphyrine, Pt(II)-méso-tétraphénylporphyrine, Pd(II)-octaéthylporphyrinecétone, Pt(II)-octaéthylporphyrinecétone Pd(II)-méso-tétra(pentafluorophényle) porphyrine, Pt(II)-mésotétra(pentafluorophényle)porphyrine, Ru(II)-tris(4,7-diphényle-1,10-phénanthroline), Ru(II)-tris(1,10-phénanthroline), tris(2,2-bipyridine)ruthénium(II)chlorure hexahydrate [Ru(bpy)3], fluorescéine, iridium(III)((N-méthyle-benzoimidazol-2-yle)-7-(diéthylamino)-coumarine))2(acétyl-acétonate), rouge lumogène, macrolex fluorescence jaune, rhodamine B, rhodamine 6G, rouge texas, bleu de thymol (TB), bleu de xylénol (XB), rouge de crésol (CR), vert de bromocrésol (BG), bleu de bromothymol (BTB), 4-nitrophénol (NP), alizarine, phénolphtaléine (PP) o-crésolphtaléine (oCP), calmagite (CG), bleu de bromoxylénol (BXB), rouge de méthyle (MR), rouge de phénol (PR), rouge neutre (NR), jaune de nitrazine (NY), 3,4,5,6 tétrabromophénolsulfonephtaléine, rouge Congo (CR), 5(6)-carboxyfluorescéine, carboxynaphtofluorescéine, acide 8-hydroxypyrène-1,3,6-trisulfonique (HPTS), séminaphtorhodafluor (colorants SNARF), séminaphtofluroescéine (colorants SNAFL) ou des colorants mentionnés au préalable liés à des éthers couronnes ou dérivés.

3. Capteur opto-chimique selon la revendication 1 ou 2, **caractérisé en ce que** l'agent tensioactif est choisi parmi le groupe constitué d'agents tensioactifs anioniques à base de carboxylates, sulfonates et sulfates, tels que des carboxylates d'alkyle, des alkylbenzènesulfonates, du sodiumdodécylebenzènesulfonate, des alcansulfonates secondaires, des sulfates tels que du sodiumlaurylsulfate, du dodécylsulfate, d'agents tensioactifs non ioniques à base d'éthoxylates d'alcools gras, de propoxylates d'alcools gras, d'alkylglucosides, d'alkylpolyglucosides, d'octylphénoléthoxylates, de nonylphénoléthoxylates, de nonoxynols, d'agents tensioactifs de copolymère en bloc ou d'agents tensioactifs cationiques à base de composés d'ammonium quaternaires, tels que du bromure d'ammonium hexadécyltriméthyle, du chlorure d'ammonium distéarylediméthyle ou d'agents tensioactifs fluorés ou d'agents tensioactifs siliconés.

4. Capteur opto-chimique selon l'une quelconque des revendications 1 à 3, **caractérisé en ce qu'**est utilisé en tant qu'agent tensioactif du bromure d'ammonium hexadécyltriméthyle.

5. Capteur opto-chimique selon l'une quelconque des revendications 1 à 4, **caractérisé en ce que** la couche de capteur est recouverte d'une couche de recouvrement perméable.

6. Capteur opto-chimique selon l'une quelconque des revendications 1 à 4, **caractérisé en ce que** la couche de capteur est formée à partir de nanofibres reliées à la couche de recouvrement perméable.

7. Capteur opto-chimique selon la revendication 5 ou 6, **caractérisé en ce que** la couche de recouvrement perméable est formée à partir de membranes poreuses, de polytétrafluoroéthylène, de polyfluorure de vinylidène TF, d'acétate de cellulose, de nitrate de cellulose ou de polyamide avec une taille de pore entre 0,1 µm et 2000 µm.

8. Capteur opto-chimique selon l'une quelconque des revendications 1 à 7, **caractérisé en ce que** la couche de capteur est formée à partir d'un polymère dopé avec de la Pt(II)-tétra(pentafluorophényle)porphyrine (PtPFTT) et mélangé à du bromure d'ammonium hexadécyletriméthyle.

9. Capteur opto-chimique selon l'une quelconque des revendications 1 à 8, **caractérisé en ce que** la couche de capteur est formée à partir de deux couches superposées l'une au-dessus de l'autre de nanofibres dopées avec des colorants luminescents différents les uns des autres.

10. Capteur opto-chimique selon l'une quelconque des revendications 1 à 8, **caractérisé en ce que** la couche de capteur est formée à partir de nanofibres contenant deux colorants luminescents différents l'un de l'autre.

11. Capteur opto-chimique selon l'une quelconque des revendications 1 à 10, **caractérisé en ce que** l'épaisseur de couche de chaque couche dopée avec un colorant luminescent, constituée de nanofibres est égale à au moins l'épaisseur simple d'une nanofibres, donc 50 nm à 1000 nm.

12. Capteur opto-chimique selon l'une quelconque des revendications 1 à 11, **caractérisé en ce que** des couches de nanofibres disposées les unes au-dessus des autres, dopées avec des colorants luminescents présentent des épaisseurs de couche différentes les unes des autres.

13. Capteur opto-chimique selon l'une quelconque des revendications 1 à 12, **caractérisé en ce que** le capteur est disposé dans un élément de serrage ou de maintien disposé dans un cache de montage.

14. Capteur opto-chimique selon l'une quelconque des revendications 1 à 13, **caractérisé en ce que** la couche de recouvrement est réalisée de manière collée au support dans la zone de l'élément de serrage ou de maintien.

15. Utilisation d'une couche de capteur appliquée sur un support, formée à partir de nanofibres tissées dopées avec un colorant luminescent, dont l'émissivité peut être modifiée après excitation avec un rayonnement électromagnétique par des substances à détecter, en tant qu'un capteur opto-chimique pour O₂, CO₂, lactate, glucose, NH₃, oxydes d'azote et ions ou des grandeurs de mesure à déterminer, choisies parmi la valeur pH, l'humidité et la température dans une phase gazeuse ou liquide, avec un temps de réponse t₉₅ inférieur à 1 s,
dans laquelle la couche de capteur est formée à partir de nanofibres avec un diamètre entre 50 nm et 1000 nm,
dans laquelle les nanofibres comprennent un polymère choisi parmi le groupe constitué de polymères à base de polysaccharide, choisis parmi la cellulose, l'acétate de cellulose (CA), le chitosane, la carboxyméthylcellulose (CMC), l'éthylcellulose, la triméthylsilylcellulose (TMSC) ou l'acide hyaluronique ; le copolymère d'éthylène-alcool vinylique (EVOH), le polyvinyle de butyral (PVB), les polyamides (PA), l'acide polyacrylique (PAA), le polyacrylonitrile (PAN), l'oxyde de polyéthylène (PEO), le polyoxyméthylène (POM), le polyester choisi parmi le téréphtalate de polyéthylène et de butylène (PET, PBT) ou le téréphtalate de polytriméthylène ; les polylactides (PLA) et les copolymères de polylactide-polyglyolide (PLGA), les polyacrylates, choisis parmi le méthacrylate de polyméthyle (PMMA) ou les polyacrylamides, les polyglycols, le polysulfone (PSU), le polyétherimide (PEI), les polythiophènes, la polyaniline (PANi), les polysilanes, les polyimides, les copolymères d'éthyle-vinyle-acétate (EVAc), le polystyrène (PS), les polyuréthanes (PU), l'alcool de polyvinyle (PVOH), le chlorure de polyvinyle (PVC), le fluorure de polyvinylidène (PVDF) ainsi que les copolymères fluorés, la polyamine, la polyvinylpyrrolidone (PVP), les polycarbonates (PC) ou les mélanges de ceux-ci,
dans laquelle les nanofibres sont dopées avec au moins un colorant luminescent choisi parmi le groupe de la métallo-porphyrine, la benzoporphyrine, la naphtoporphyrine, la phtallocyanine, le pérylènediimine, le pyrène ; les colorants du xanthène, les colorants azoïques, les colorants bodipy, la bipyridine, la phénantroline, la coumarine et l'acétylacétonate de ruthénium et d'iridium ; les colorants d'acridine, les colorants d'oxazine, la coumarine, l'azaannulène, la squarine, la 8-hydroxyquinoléine, les nanoparticules luminescentes, choisies parmi des points quantiques ou des nanocristaux ; le carbostyryle, les complexes de terbium, les phosphores inorganiques ou les colorants liés à des ionophores ou dérivés des classes mentionnées plus haut, et
dans lequel les nanofibres contiennent par ailleurs un agent tensioactif anionique, cationique ou non ionique.
